# DEMANDE DE BREVET EUROPEEN

(11) **EP 1 502 575 A1**
(43) Date de publication de la demande: **02.02.2005**
(21) Numéro de dépôt: 04300500.8
(22) Date de dépôt: 30.07.2004
(51) Int. Cl.: A61K 7/02

(54) **Composition cosmétique colorée à effet de flou**

(30) Priorité: 01.08.2003 FR 0309572
(71) Demandeur: L'OREAL, 75008 Paris (FR)
(72) Inventeur: Chevalier, Gaetan, 45760, BOIGNY SUR BIONNE (FR); Girier-Dufournier, Franck, 75011, PARIS (FR)
(74) Mandataire: Tanty, François

(57) **Abrégé**

La présente invention concerne une composition cosmétique colorée comportant, dans un milieu physiologiquement acceptable, une charge et un agent de coloration, cet agent de coloration présentant une transparence et/ou étant dans une quantité telle que sa présence avec la charge dans la composition confère à celle-ci une saturation C* supérieure ou égale à 17, un facteur de transmission hémisphérique supérieur ou égal à 70 % et un indice de flou H supérieur ou égal à 40 %.

## Description

La présente invention se rapporte aux compositions cosmétiques destinées au traitement de la peau, des muqueuses ou des phanères, notamment des matières kératiniques non fibreuses.

Il existe un besoin pour bénéficier d'une composition capable d'apporter de la couleur et de camoufler des défauts, de la peau notamment, tout en permettant à la surface traitée de conserver un aspect naturel.

L'invention vise notamment à répondre à ce besoin.

L'invention a ainsi pour objet, selon un premier de ses aspects, une composition cosmétique colorée, comportant, dans un milieu physiologiquement acceptable, une charge et un agent de coloration, cet agent de coloration présentant une transparence et/ou étant dans une quantité telle que sa présence dans la composition avec la charge confère à celle-ci une saturation C* supérieure ou égale à 17, un facteur de transmission hémisphérique Th supérieur ou égal à 70 % et un indice de flou supérieur ou égal à 40 %.

Une telle composition, notamment lorsqu'elle est appliquée sur la peau, peut permettre de masquer des imperfections de celle-ci et d'apporter de la couleur sans pour autant rendre le maquillage particulièrement apparent. Un aspect relativement naturel du support maquillé peut ainsi être conservé.

### Mesure du facteur de transmission hémisphérique Th et calcul de l'indice de flou H

Comme indiqué ci-dessus, la composition cosmétique selon l'invention présente un indice de flou H supérieur ou égal à 40 %, de préférence supérieur ou égal à 50 %, voire à 60 %, mieux supérieur ou égal à 70 % et mieux encore supérieur ou égal à 80%.

Le facteur de transmission hémisphérique Th est quant à lui supérieur ou égal à 70 %, mieux supérieur ou égal à 80 %, voire à 85 %.

On désigne par « indice de flou H » la grandeur ((Th - Td)/Th).100, dans laquelle Th représente le facteur de transmission hémisphérique et Td le facteur de transmission directe.

Lorsque l'indice de flou H est élevé, la composition procure un effet de flou important, permettant de masquer des défauts en changeant la perception du relief.

Le facteur de transmission hémisphérique Th donne une information sur la transparence de la composition. Plus la valeur Th est élevée, plus la composition est transparente.

Les valeurs Th et Td peuvent être mesurées à l'aide d'un spectrophotomètre et d'une sphère d'intégration, placée derrière la composition que l'on cherche à caractériser.

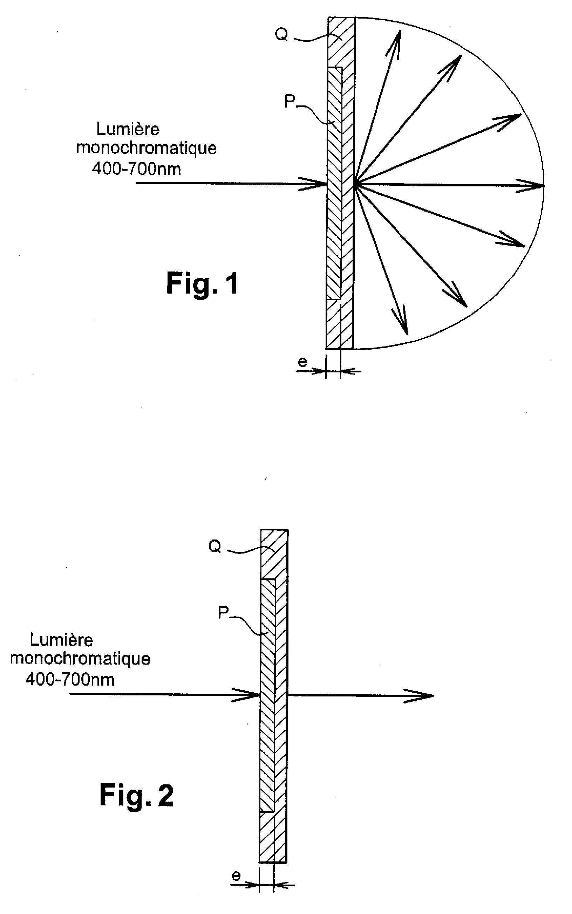

La transmission hémisphérique spectrale Th(λ) de la composition est définie par le rapport entre l'intensité lumineuse de longueur d'onde λ reçue par un échantillon de composition P et l'intensité lumineuse restituée par cet échantillon dans toutes les directions d'un espace délimité par un plan, comme illustré à la figure 1.

La transmission directe spectrale Td(λ) d'un échantillon est définie par le rapport entre l'intensité lumineuse de longueur d'onde λ reçue par l'échantillon et l'intensité lumineuse restituée par cet échantillon dans la même direction de propagation que la lumière incidente, comme illustré à la figure 2.

Les mesures des transmissions spectrales hémisphérique Th(λ) et directe Td(λ) peuvent être effectuées selon le protocole expérimental suivant.

La composition P que l'on cherche à analyser est étalée sur une lame de quartz Q creusée, de manière à former une couche d'une épaisseur *e* de 20 µm, puis placée dans une étuve pendant 5 minutes à 37 °C.

La valeur Th(λ) peut être mesurée à l'aide d'un spectrophotomètre VARIAN Cary 300® et d'une sphère d'intégration de marque Labsphère® placée à l'arrière de la lame de quartz contenant la composition. Le spectrophotomètre est utilisé en mode de transmission diffuse et la longueur d'onde λ de la lumière incidente monochromatique varie de 400 à 700 nm.

La mesure est effectuée en mode transmission % T, à une vitesse de balayage de 240 nm/min et en mode « double reverse ».

Dans un premier temps, il est procédé à un étalonnage en faisant une première mesure sur la lame de quartz Q vide pour obtenir la valeur maximale de l'intensité transmise.

Ensuite, on mesure la transmission hémisphérique spectrale Th(λ) avec la lame de quartz contenant la composition P à analyser.

La valeur Td(λ) est mesurée à l'aide du même spectrophotomètre, utilisé en mode transmission directe et la longueur d'onde λ de la lumière incidente monochromatique incidente varie de 400 à 700 nm. On se place en mode transmission % T, à une vitesse de balayage 240 nm/min, en mode « double ». On place une lame de quartz vide dans un compartiment de référence et la lame de quartz contenant la composition est placée dans un compartiment de mesure, puis Td(λ) est mesuré.

Les facteurs de transmission hémisphérique Th et directe Td sont calculés en établissant respectivement la moyenne de toutes les valeurs spectrales Th(λ) ou Td(λ) obtenues lorsque la longueur d'onde varie de 400 à 700 nm.

### Saturation C*

La saturation C* peut être mesurée dans l'espace colorimétrique CIE L*a*b*C*h, de la façon suivante.

La composition est déposée à ras bord d'une coupelle en acier galvanisé et présentant une profondeur de 2,47 mm minimum.

La coupelle est ensuite recouverte d'une lame porte-objet « LMR » de type H1, à bords biseautés, de dimensions 76 x 26 mm, de la société LABO-MODERNE.

La saturation C* est mesurée au moyen d'un spectrocolorimètre CM3700d, le système d'éclairage et d'observation étant en mode réflectance d/8°, les mesures étant effectuées en mode réflexion spéculaire exclue.

L'émission UV est 100 % incluse.

La position zoom est moyenne (MAV), l'ouverture est moyenne, la configuration est CREIMM, l'observation est 10° CIE 1964, l'illuminant est D65.

La saturation C* de la composition, mesurée de la sorte, est supérieure ou égale à 17, mieux supérieure ou égale à 20, et de préférence inférieure ou égale à 60.

Une valeur supérieure à 17 permet d'obtenir un apport de couleur suffisant tandis qu'une valeur inférieure à 60 permet de ne pas nuire à l'effet de flou et de conserver un maquillage naturel, le cas échéant.

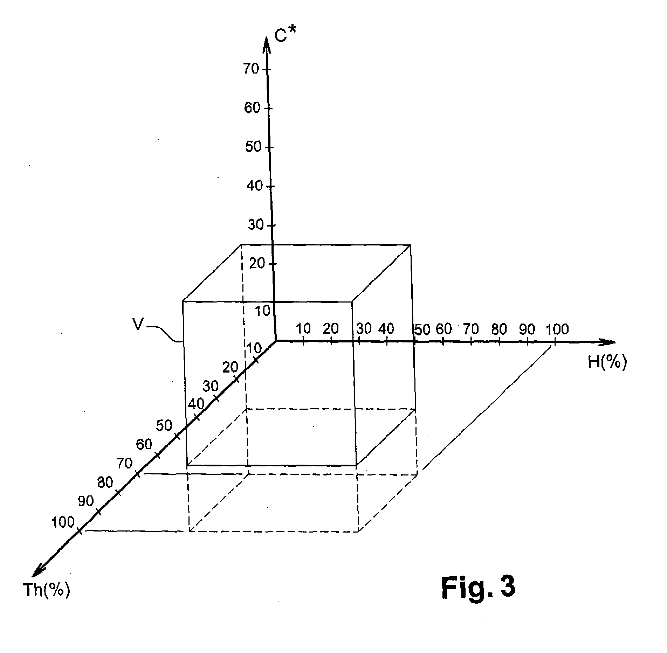

La figure 3 représente dans l'espace Th, H, C* un exemple de domaine privilégié V des valeurs Th, H et C* d'une composition selon l'invention.

### Agent de coloration

Par « agent de coloration », on désigne au sens de la présente invention tout pigment ou colorant ou mélange de pigments et/ou de colorants propre à apporter suffisamment de couleur à la composition pour lui permettre de présenter la saturation C* requise tout en permettant d'obtenir les valeurs d'indice de flou H et de facteur de transmission hémisphérique Th recherchées.

L'agent de coloration peut notamment comporter ou être constitué de particules d'au moins un pigment. Les particules de pigment peuvent avoir subi, le cas échéant, un traitement ayant pour objectif d'augmenter la stabilité de la couleur et faciliter leur incorporation dans la composition. En particulier, des particules de pigment traitées en vu de les rendre hydrophobes seront plus facilement dispersibles dans une phase huileuse, par exemple.

Les particules de pigment peuvent présenter des formes diverses, notamment une forme sensiblement sphérique ou aplatie.

Les particules de pigment peuvent présenter une structure multicouche, et notamment un coeur transparent, par exemple en silice.

De telles particules de pigment à coeur transparent permettent de ne pas opacifier outre mesure la composition, ce qui est favorable à l'obtention des valeurs de H et Th souhaitées

Le pigment peut être non interférentiel et/ou non fluorescent.

Dans le cas notamment d'un pigment dont les particules présentent une structure multicouche relativement transparente, la proportion de pigment peut être relativement élevée, étant par exemple supérieure ou égale à 1 % en poids, notamment comprise entre 1,5 et 10 % en poids, mieux entre 2 % et 8 % en poids, par rapport au poids total de la composition. La proportion peut par exemple être comprise entre 2 % et 5 %, étant par exemple de l'ordre de 3 %.

Plus la transparence du pigment est élevée, plus la quantité de pigment pourra, a priori, être importante sans faire perdre à la composition l'effet de flou et le facteur de transmission hémisphérique recherchés.

La transparence du pigment peut être quantifiée par son rapport de contraste, défini plus loin, celui-ci étant compris par exemple dans le cas de pigments tels que ci-dessus entre 15 et 65, mieux inférieur ou égal à 50. Le rapport de contraste peut notamment être inférieur ou égal à 45, mieux inférieur ou égal à 40, ou mieux encore 35.

L'agent de coloration peut comporter un pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

L'agent de coloration peut encore comporter un pigment ayant une structure qui peut être par exemple de type microsphères de silice contenant de l'oxyde de fer. Un exemple de pigment présentant cette structure et un rapport de contraste relativement faible est celui commercialisé par la société MIYOSHI sous la référence PC BALL PC-LL-100 P, ce pigment étant constitué de microsphères de silice contenant de l'oxyde de fer jaune.

Des valeurs relativement faibles de rapport de contraste, combinées aux valeurs d'indice de flou et de facteur de transmission hémisphérique, permettent d'obtenir des résultats particulièrement satisfaisants en termes d'apport conjoint de couleur et de couvrance, tout en gardant un effet de flou important, permettant de masquer les imperfections.

Dans le cas d'un mélange de pigments, les proportions de ceux-ci pourront être ajustées en fonction de leur rapport de contraste.

La composition peut être dépourvue de pigments de rapport de contraste supérieur à 60.

La composition peut encore, en variante, comporter des particules d'au moins un pigment qui est relativement opaque, notamment qui soit de rapport de contraste supérieur à 60, sous réserve que la quantité introduite dans la composition ne nuise pas à l'obtention des valeurs de H et Th recherchées.

La composition peut ainsi comporter, par exemple, des pigments conventionnels ayant un rapport de contraste relativement élevé, par exemple supérieur ou égal à 40, par exemple des pigments du type oxydes de fer et/ou dioxyde de titane.

Les pigments peuvent avoir un coeur non transparent.

La composition comporte de préférence moins de 2 % en poids de tels pigments relativement opaques, mieux moins de 1,5 %, mieux encore moins de 1 %, mieux encore moins de 0,75 %, par exemple entre 0,25 et 0,75 %, par exemple de l'ordre de 0,5 %, par rapport au poids total de la composition.

L'agent de coloration peut encore être choisi parmi les colorants, notamment les colorants hydrosolubles ou liposolubles ou d'autres matières colorantes encore, dans la mesure où leur incorporation à la composition ne nuit pas à l'obtention de l'indice de flou H et du facteur de transmission hémisphérique Th recherchés. L'agent de coloration peut ainsi comporter un colorant hydrosoluble tel que du caramel, par exemple

L'agent de coloration peut avoir une couleur correspondant à celle de la peau sur laquelle il est destiné à être appliqué, notamment comporter au moins un pigment brun, jaune ou rouge.

L'agent de coloration peut être dépourvu de nacres.

### Mesure du rapport de contraste

Le rapport de contraste, encore appelé « contrast ratio » se trouve défini notamment dans la demande internationale WO 98/52534.

Pour calculer le rapport de contraste, on procède de la manière suivante.

Un mélange formé de 5 % en poids, par rapport au poids total, de l'agent de coloration à étudier et pour le reste de l'émulsion de référence ci-dessous est appliquée sur un support opaque noir et sur un support opaque blanc, selon un film d'une épaisseur de 50 µm. Ce film est séché pendant 24 heures à 25° C +/- 1 °C sous une pression de 1 atm.

L'émulsion de référence a pour formulation :

| | % en poids |
|---|---|
| - Eau | 45,83 |
| - p-hydroxybenzoate de méthyle | 0,45 |
| - Chlorphénesin | 0,34 |
| - Disodium EDTA | 0,11 |
| - Glycérine | 5,62 |
| - PEG-8 | 2,25 |
| - PEG-20 | 1,12 |
| - Silicate de magnésium et d'aluminium | 0,9 |
| - Lauroyl sarcosinate de sodium | 1,68 |
| - Dioxyde de titane (et) alumine (et) glycérine (et) silice | 3,37 |
| - Triéthanolamine | 1,35 |
| - Acide stéarique | 2,7 |
| - Stéarate de glycéryle | 2,02 |
| - p-hydroxybenzoate de butyle | 0,17 |
| - Isononanoate d'isononyle | 8,99 |
| - Cyclohexasiloxane | 6,57 |
| - Diméthicone | 10,28 |
| - BIS-PEG-15 méthyléther diméthicone | 2,25 |
| - Talc | 1,12 |
| - Kaolin | 1,12 |
| - Polyméthyl méthacrylate | 1,69 |

A l'aide d'un colorimètre, par exemple du type MINOLTA CR-200, en mode illuminant D65, angle de vue 0°, on mesure la valeur du tristimulus Y de la composition en trois endroits différents du support noir et en trois endroits différents du support blanc.

Le rapport de contraste correspond à la moyenne des trois valeurs de Y mesurées sur le support noir, divisée par la moyenne des trois valeurs de Y mesurées sur le support blanc, et multipliée par 100.

Plus le rapport de contraste est élevé et proche de 100 %, plus l'agent de coloration est opaque. Plus le rapport de contraste est faible, plus l'agent de coloration est transparent.

### Charges

Les charges considérées peuvent être de toute nature chimique dans la mesure où elles sont compatibles avec une utilisation en cosmétique et où elles n'affectent pas les propriétés attendues de la composition selon l'invention.

La composition selon l'invention comporte au moins une charge dite à effet de flou, contribuant à donner à la composition, en présence de l'agent de coloration, l'indice de flou H et le facteur de transmission hémisphérique Th souhaités.

La charge à effet de flou peut être sensiblement incolore au sein de la composition.

Les charges à effet de flou susceptibles d'être utilisées dans la composition selon l'invention peuvent notamment présenter une taille moyenne en nombre inférieure ou égale à 15 µm, notamment inférieure ou égale à 10 µm, en particulier inférieure ou égale à 7,5 µm, par exemple de l'ordre de 1 à 5 µm.

Par « taille moyenne en nombre », on désigne la dimension donnée par la distribution granulométrique statistique à la moitié de la population, dite D50.

La charge à effet de flou utilisée peut être choisie notamment parmi les poudres de silice et silicates, notamment d'alumine, les poudres de polyméthylméthacrylate, le talc, les composites silice/TiO₂ ou silice/oxyde de zinc, les poudres de polyéthylène, les poudres d'amidon, les poudres de polyamide, les poudres de copolymères styrène/acrylique, les élastomères de silicone, et leurs mélanges, cette liste n'étant pas limitative.

Comme exemples particuliers de charges à effet de flou, on peut citer :
- le talc de taille moyenne inférieure ou égale à 3 µm, notamment celui vendu sous la dénomination commerciale Talc P3® par la société Nippon Talc,
- la poudre de Nylon® 12, notamment celle vendue sous le dénomination Orgasol 2002 Extra D Nat Cos® par la société Atochem,
- les micro-billes de silice telles que celles vendues sous la dénomination SB-700® ou SB-150® par la société Miyoshi,
- les microsphères de silice amorphe, par exemple de référence SUNSPHERE H-53, de taille moyenne en nombre 5 µm, de la société ASAHI GLASS,
- les particules de silice, traitées en surface par une cire minérale (1 à 2 %) de la société DEGUSSA.

La charge à effet de flou peut être présente dans la composition cosmétique selon l'invention avec une teneur allant de préférence de 0,1 à 20 % en poids, et notamment allant de 1 % à 12 % en poids par rapport au poids total de la composition, notamment encore entre 5 et 10 % en poids, par exemple de l'ordre de 8 % en poids, la proportion étant choisie notamment en fonction de la nature et/ou de la quantité de l'agent de coloration et de l'effet recherché.

### Milieu physiologiquement acceptable

La composition selon l'invention comprend un milieu physiologiquement acceptable.

Par « milieu physiologiquement acceptable », on désigne un milieu non toxique et susceptible d'être appliqué sur la peau, notamment celle du visage et/ou du cou, les muqueuses, notamment les lèvres, ou les phanères, notamment les ongles, d'êtres humains. Le milieu physiologiquement acceptable est généralement adapté à la nature du support sur lequel doit être appliquée la composition ainsi qu'à la forme sous laquelle la composition est conditionnée.

La composition selon l'invention peut être une émulsion. La proportion de la phase grasse de la composition peut aller par exemple de 5 à 80 % en poids, et notamment de 5 à 50 % en poids, par rapport au poids total de la composition.

La composition peut comprendre par exemple au moins un milieu aqueux, constituant une phase aqueuse formant la phase continue de la composition.

La phase aqueuse peut être constituée essentiellement d'eau. Toutefois, elle peut également comprendre un mélange d'eau et de solvant organique miscible à l'eau, notamment de miscibilité dans l'eau supérieure à 50 % en poids à 25 °C, comme les monoalcools inférieurs ayant de 1 à 5 atomes de carbone tels que par exemple l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

Cette phase aqueuse, comportant notamment de l'eau et éventuellement un solvant organique miscible à l'eau, peut être présente dans la composition en une teneur allant par exemple de 1 % à 95 % en poids, notamment allant de 3 % à 80 % en poids, et en particulier allant de 5 % à 60 % en poids, par rapport au poids total de la composition.

La composition peut également comprendre une phase grasse, par exemple.

En particulier, la phase grasse de la composition peut comprendre par exemple au moins un huile, notamment une huile choisie parmi :
- les huiles hydrocarbonées d'origine animale, telles que le perhydrosqualène,
- les huiles hydrocarbonées d'origine végétale, telles que les triglycérides liquides d'acides gras comportant de 4 à 10 atomes de carbone et la fraction liquide du beurre de karité,
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle,
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam,
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique,
- les huiles fluorées partiellement hydrocarbonées et/ou siliconées comme celles décrites dans le document JP-A-2-295912,
- les huiles de silicone comme les polyméthylsiloxanes (PDMS) volatiles ou non, à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes, et
- leurs mélanges.

La composition peut également comprendre, par exemple, au moins une cire, au moins une gomme et/ou au moins un corps gras pâteux, d'origine végétale, animale, minérale ou de synthèse, siliconé ou non.

Les cires peuvent être hydrocarbonées, siliconées et/ou fluorées, comportant éventuellement des fonctions ester ou hydroxyle. Elles peuvent notamment être d'origine naturelle.

La cire peut représenter de 0,01 à 10 % en poids, notamment de 0,1 à 5 % en poids, par rapport au poids total de la composition. La composition peut aussi être exempte de cires.

La composition peut comprendre au moins un polymère filmogène, par exemple. On désigne par « polymère filmogène » un polymère apte à former à lui seul ou en présence d'un agent auxiliaire de filmification, un film continu et adhérent sur la peau.

Le polymère filmogène être par exemple au moins un polymère choisi parmi le groupe comprenant :
- les polymères filmogènes hydrosolubles,
- les dispersions aqueuses de particules de polymères filmogènes hydrodispersibles, encore appelées « latex » ; dans ce cas, la composition doit comprendre une phase aqueuse,
- les polymères filmogènes liposolubles,
- les polymères filmogènes lipodispersibles sous forme de dispersions non aqueuses de particules de polymère, de préférence des dispersions de particules polymériques, le cas échéant stabilisées en leur surface par au moins un agent stabilisant, dans une ou plusieurs huiles de silicones et/ou hydrocarbonées ; ces dispersions non aqueuses sont encore appelées « NAD », et
- leurs mélanges.

Comme dispersions aqueuses de polymère filmogène utilisables selon l'invention, on peut citer les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou bien encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère filmogène, on peut également citer les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Comme exemples de polymères filmogènes hydrosolubles, on peut citer les protéines comme les protéines d'origine végétale, les polymères de chitine ou de chitosane anioniques, cationiques, amphotères ou non-ioniques, les polymères de cellulose, les polymères ou copolymères acryliques, les polymères vinyliques, les polymères d'origine naturelle, éventuellement modifiés, et leurs mélanges.

Des dispersions de polymère filmogène dans la phase grasse liquide, en présence d'agent stabilisants, sont notamment décrites dans les documents EP-A-749746, EP-A-923928, EP-A-930060.

A titre d'exemple de polymères liposolubles, on peut citer par exemple les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Le polymère filmogène peut être présent dans la composition par exemple en une teneur en matières sèches allant de 0,01 à 20 % en poids et notamment de 0,5 % à 10 % en poids par rapport au poids total de la composition.

La composition peut contenir également les adjuvants habituels dans le domaine cosmétique, tels que les émulsionnants, les gélifiants hydrophiles ou lipophiles, les actifs hydrophiles ou lipophiles, les conservateurs, les antioxydants, les solvants, les parfums, les absorbeurs d'odeur et des ajusteurs de pH.

Les quantités de ces différents adjuvants sont celles classiquement utilisées dans le domaine considéré, et représentent par exemple de 0,01 à 20% du poids total de la composition. Ces adjuvants, selon leur nature, peuvent être introduits dans une phase grasse ou dans une phase aqueuse.

En tout état de cause, ces adjuvants, ainsi que leurs proportions, seront choisis de manière à ne pas nuire aux propriétés optiques recherchées.

Les agents tensioactifs peuvent être choisis parmi des agents tensioactifs anioniques ou non ioniques. On peut se reporter par exemple au document « Encyclopedia of Chemical Technology, KIRK-OTHMER », volume 22, p.333-432, 3ème édition, 1979, WILEY, pour la défmition des propriétés et des fonctions des tensioactifs, en particulier p.347-377 de cette référence, pour les tensioactifs anioniques et non-ioniques.

Les tensioactifs utilisés peuvent être choisis par exemple :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.

Comme émulsionnants et co-émulsionnants, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que ceux disponibles sous les dénominations commerciales ABIL WE09, ABIL EM90 et ABIL EM97 auprès de la société Degussa Goldschmidt ou le mélange de stéarate d'éthylène glycol acétyle et de tristéarate de glycéryle commercialisé par la société Guardian sous la dénomination commerciale UNITWIX.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme agents actifs habituels dans le domaine cosmétique ou dermatologique pouvant être utilisés, on peut citer en particulier tous les actifs connus pour leur activité sur le vieillissement de la peau comme les agents kératolytiques ou prodesquamants, par exemple les α-hydroxy-acides, les β-hydroxy-acides, les α-céto-acides, les β-ceto-acides, les rétinoïdes et leurs esters, le rétinal, l'acide rétinoïque et ses dérivés. On peut citer aussi les extraits de plantes veinotoniques tels que les extraits de petit houx et/ou de marron d'Inde ; les bases xanthiques telles que la caféine ; les vitamines, telles que par exemple les vitamines A, B3, PP, B5, E, K1 et/ou C et les dérivés de ces vitamines et notamment leurs esters ; les agents anti-radicaux libres ; les filtres solaires ; les agents hydratants comme les polyols ; les céramides ; la DHEA et ses dérivés ; le coenzyme Q10 ; les agents blanchissants et dépigmentants comme l'acide kojique, les extraits de scutellaire, de mûrier, de réglisse et/ou de camomille ; les dérivés de para-aminophénols, l'arbutine et leurs dérivés, et leurs mélanges.

Pour une utilisation dans le traitement cosmétique des peaux grasses ou mixtes, la composition selon l'invention peut contenir au moins un actif choisi par exemple parmi : les vitamines B3 et B5 ; les sels de zinc, et en particulier l'oxyde de zinc et le gluconate de zinc ; l'acide salicylique et ses dérivés tels que l'acide n-octanoyl-5-salicylique ; le triclosan ; la capryloylglycine ; un extrait de clou de girofle ; l'octopirox ; l'hexamidine ; et l'acide azélaïque et ses dérivés.

On peut également introduire dans la composition des filtres UVA et/ou UVB, choisis parmi les filtres organiques et les filtres minéraux éventuellement enrobés pour les rendre hydrophobes.

La composition selon l'invention peut se présenter sous toutes les formes galéniques normalement utilisées dans le domaine cosmétique, et elle peut être notamment sous forme d'une solution huileuse éventuellement gélifiée, d'une émulsion obtenue par dispersion d'une phase grasse dans une phase aqueuse (H/E) ou inversement (E/H), d'une émulsion triple (E/H/E ou H/E/H), d'une dispersion vésiculaire de type ionique (liposomes ou oléosomes) et/ou non ionique (niosomes) et/ou d'une dispersion de nanocapsules ou nanosphères.

La composition selon l'invention peut être plus ou moins fluide et avoir l'aspect d'une crème, d'une pommade, d'un lait, d'une lotion, d'un sérum, d'une pâte, d'une mousse, d'un gel, par exemple. Elle peut également se présenter sous forme solide, en particulier sous forme de stick ou de coupelle ou sous d'autres formes galéniques encore, notamment pulvérulente. Eventuellement, la composition peut présenter une consistance adaptée à son application par pulvérisation.

L'invention a encore pour objet un fond de teint à effet de flou selon l'invention.

L'invention a encore pour objet l'utilisation d'une composition à effet de flou selon l'invention pour le maquillage de la peau, des muqueuses ou des ongles.

Les exemples de formulation figurant ci-après sont présentés à titre purement illustratif, les pourcentages étant tous exprimés en poids par rapport au poids total de la composition.

### Exemple 1

| | % en poids |
|---|---|
| - Polyglycéryl-4 isostéarate (et) laurate d'hexyle (et) cétyl PEG/PPG-10/1 diméthicone | 9 |
| - Stéarate de glycol acétylé | 0,7 |
| - p-hydroxybenzoate de butyle | 0,15 |
| - 2-oléamido-1,3-octadécanediol | 0,04 |
| - Cyclopentasiloxane (et) distéardimonium hectorite (et) alcool dénat. | 8 |
| - Cyclopentasiloxane | 14,25 |
| - Diméthicone | 4 |
| - Isododécane | 2,6 |
| - Pigment COVERLEAF NS de la société CHEMICALS AND CATALYSTS | 3 |
| - Talc P3 de la société NIPPON TALC | 8 |
| - Eau | 44,06 |
| | |
| - Sulfate de magnésium | 0,7 |
| - p-hydroxybenzoate de méthyle | 0,25 |
| - Propylène glycol | 5 |
| - Chlorphénesine | 0,25 |

On mesure les valeurs suivantes :
Th ≥ 90 %
H ≥ 75 %
C* ≈ 20

### Exemple 2

| | % en poids |
|---|---|
| - Polyglycéryl-4 isostéarate (et) laurate d'hexyle (et) cétyl PEG/PPG-10/1 diméthicone | 9 |
| - Stéarate de glycol acétylé | 0,7 |
| - p-hydroxybenzoate de butyle | 0,15 |
| - 2-Oléamido-1,3-octadécanediol | 0,04 |
| - Cyclopentasiloxane (et) distéardimonium hectorite (et) alcool dénat. | 8 |
| - Cyclopentasiloxane | 14,25 |
| - Diméthicone | 4 |
| - Isododécane | 2,6 |
| - Oxydes de fer (et) glutamate de stéaroyle disodium (et) hydroxyde d'aluminium | 0,18 |
| - Dioxyde de titane (et) glutamate de stéaroyle disodium (et) hydroxyde d'aluminium | 0,32 |
| - Talc | 8 |
| - Eau | 46,56 |
| - Sulfate de magnésium | 0,7 |
| - p-hydroxybenzoate de méthyle | 0,25 |
| - Propylène glycol | 5 |
| - Chlorphénesine | 0,25 |

On mesure les valeurs suivantes :
Th ≥ 80 %
H ≥ 80 %
C* ≈ 25

On observe que ces deux compositions permettent d'atténuer les défauts de la peau, tout en apportant de la couleur et en conférant un maquillage naturel.

Le contraste entre les zones d'ombre et les zones éclairées est réduit.

Dans toute la description, y compris les revendications, l'expression « comportant un » doit être comprise comme étant synonyme de « comportant au moins un », sauf si le contraire est spécifié.

L'expression « compris entre » signifie bornes incluses, sauf si le contraire est spécifié.

## Revendications

1. Composition cosmétique colorée comportant, dans un milieu physiologiquement acceptable, une charge et un agent de coloration, cet agent de coloration présentant une transparence et/ou étant dans une quantité telle que sa présence avec la charge dans la composition confère à celle-ci une saturation C* supérieure ou égale à 17, un facteur de transmission hémisphérique supérieur ou égal à 70 % et un indice de flou H supérieur ou égal à 40 %.

2. Composition selon la revendication 1, **caractérisée par le fait que** l'indice de flou H est supérieur ou égal à 50 %, de préférence à 60 %, mieux supérieur ou égal à 70%.

3. Composition selon la revendication 1, **caractérisée par le fait que** l'indice de flou H est supérieur ou égal à 80 %.

4. Composition selon l'une quelconque des revendications précédentes, **caractérisée par le fait que** le facteur de transmission hémisphérique Th est compris entre 80 % et 100 %.

5. Composition selon l'une quelconque des revendications précédentes, **caractérisée par le fait que** le facteur de transmission hémisphérique Th est compris entre 85 % et 100 %.

6. Composition selon l'une quelconque des revendications 1 à 5, **caractérisée par le fait que** la charge est choisie dans le groupe constitué par les poudres de silice et silicates, notamment d'alumine, les poudres de polyméthylméthacrylate, le talc, les composites silice/TiO₂ ou silice/oxyde de zinc, les poudres de polyéthylène, les poudres d'amidon, les poudres de polyamides, les poudres de copolymère styrène/acrylique, les élastomères de silicone, et leurs mélanges.

7. Composition selon l'une quelconque des revendications précédentes, **caractérisée par le fait que** la charge est constituée de particules dont la taille moyenne en nombre est inférieure ou égale à 15 µm.

8. Composition selon la revendication 7, **caractérisée par le fait que** la taille moyenne en nombre des particules de la charge est inférieure ou égale à 10 µm.

9. Composition selon la revendication 7, **caractérisée par le fait que** la taille moyenne en nombre des particules de la charge est inférieure ou égale à 7,5 µm.

10. Composition selon l'une quelconque des revendications précédentes, **caractérisée par le fait que** la charge est dans une proportion comprise entre 0,1 % et 20 % en poids, notamment entre 1 % et 12 % en poids, par rapport au poids total de la composition, mieux entre 5 et 10 % en poids.

11. Composition selon l'une quelconque des revendications précédentes, **caractérisée par le fait que** la charge est dans une proportion d'environ 8 % en poids par rapport au poids total de la composition.

12. Composition selon l'une quelconque des revendications précédentes, **caractérisée par le fait que** l'agent de coloration comporte des particules d'au moins un pigment.

13. Composition selon la revendication 12, **caractérisée par le fait que** les particules de pigment présentent une structure multicouche.

14. Composition selon la revendication 13, **caractérisée par le fait que** les particules de pigment comportent un coeur transparent.

15. Composition selon la revendication 14, **caractérisée par le fait que** le coeur est en silice.

16. Composition selon l'une quelconque des revendications 12 à 15, **caractérisée par le fait que** le pigment est non interférentiel.

17. Composition selon l'une quelconque des revendications 12 à 16, **caractérisée par le fait que** le pigment est non fluorescent.

18. Composition selon l'une quelconque des revendications 12 à 17, **caractérisée par le fait que** la proportion de pigment est supérieure ou égale à 1 % en poids par rapport au poids total de la composition, notamment est comprise entre 1,5 et 10 %, notamment est comprise entre 2 % et 8 % en poids par rapport au poids total de la composition.

19. Composition selon l'une quelconque des revendications 12 à 17, **caractérisée par le fait que** la proportion de pigment est comprise entre 2 % et 5 % en poids par rapport au poids total de la composition.

20. Composition selon la revendication 18, **caractérisée par le fait que** la proportion de pigment est de l'ordre de 3 %.

21. Composition selon l'une quelconque des revendications 12 à 20, **caractérisée par le fait que** le pigment présente un rapport de contraste compris entre 15 et 65.

22. Composition selon l'une quelconque des revendications 12 à 20, **caractérisée par le fait que** le pigment présente un rapport de contraste inférieur ou égal à 50.

23. Composition selon la revendication 21, **caractérisée par le fait que** le rapport de contraste est inférieur ou égal à 45, mieux inférieur ou égal à 40.

24. Composition selon la revendication 21, **caractérisée par le fait que** le rapport de contraste est inférieur ou égal à 35.

25. Composition selon l'une quelconque des revendications 18 à 24, **caractérisée par le fait que** le pigment présente une structure de type séricite/oxyde de fer brun/dioxyde de titane/silice.

26. Composition selon l'une quelconque des revendications 18 à 24, **caractérisée par le fait que** le pigment présente une structure de type microsphères de silice contenant de l'oxyde de fer.

27. Composition selon l'une quelconque des revendications 18 à 24, **caractérisée par le fait que** le pigment présente une structure de type microsphères de silice contenant de l'oxyde de fer jaune.

28. Composition selon l'une quelconque des revendications précédentes, **caractérisée par le fait que** la composition est dépourvue de pigments de rapport de contraste supérieur à 60.

29. Composition selon la revendication 12, **caractérisée par le fait que** les particules de pigment présentent un coeur non transparent.

30. Composition selon la revendication 29, **caractérisée par le fait que** la composition comporte moins de 2 % en poids de pigment, mieux moins de 1,5 %, mieux encore moins de 1 % en poids par rapport au poids total de la composition.

31. Composition selon la revendication 29, **caractérisée par le fait que** la composition comporte moins de 0,7 % en poids de pigment par rapport au poids total de la composition.

32. Composition selon la revendication 29, **caractérisé par le fait que** les particules de pigment présentent un rapport de contraste supérieur à 40.

33. Composition selon la revendication 29, **caractérisée par le fait que** la composition comporte entre 0,25 % et 0,75 % en poids de pigment, par rapport au poids total de la composition.

34. Composition selon la revendication 29, **caractérisée par le fait que** la composition comporte de l'ordre de 0,5 % en poids de pigment par rapport au poids total de la composition.

35. Composition selon l'une quelconque des revendications 29 à 34, **caractérisée par** le fait l'agent de coloration comporte des particules d'un pigment de type oxydes de fer et/ou dioxyde de titane.

36. Composition selon l'une quelconque des revendications précédentes, **caractérisée par le fait que** la saturation C* est supérieure ou égale à 20.

37. Composition selon l'une quelconque des revendications précédentes, **caractérisée par le fait que** la saturation C* est inférieure ou égale à 60.

38. Composition selon l'une quelconque des revendications 1 à 37, **caractérisé par le fait que** l'agent de coloration comporte un colorant hydrosoluble.

39. Composition selon l'une quelconque des revendications 1 à 37, **caractérisé par le fait que** le colorant hydrosoluble comporte du caramel.

40. Fond de teint comportant une composition telle que définie dans l'une quelconque des revendications 1 à 39.

41. Utilisation de la composition telle que définie dans l'une quelconque des revendications 1 à 39 pour le maquillage de la peau, des ongles ou des muqueuses.
